# EUROPEAN PATENT APPLICATION

(11) **EP 1 127 582 A2**
(43) Date of publication of application: **29.08.2001**
(21) Application number: 01301671.2
(22) Date of filing: 23.02.2001
(51) Int. Cl.: A61L 31/16, A61F 2/06

(54) **Use of cladribine on a stent to prevent restenosis**

(30) Priority: 25.02.2000 US 512432
(71) Applicant: Cordis Corporation, Miami Lakes Florida 33014 (US)
(72) Inventor: Kopia, Gregory A., Neshanic, New Jersey 08853 (US); Falotico, Robert, Belle Mead, New Jersey 08502 (US)
(74) Representative: Fisher, Adrian John

(57) **Abstract**

The current invention comprises an approach to solving the clinical problem of restenosis, which involves the administration of the antiproliferative antineoplastic agent, cladribine, to patients undergoing PTCA or stent implantation. In one embodiment of the invention, cladribine is administered to patients systemically, either subcutaneously or intravenously. In another embodiment of the invention, cladribine is bound to the surface of a stent by means of incorporation within either a biodegradable or biostable polymeric coating. Alternatively, cladribine could be incorporated into a stent constructed with a grooved reservoir.

## Description

### Field of the Invention:

This invention describes the delivery of cladribine either systemically or locally, particularly from an intravascular stent, directly from micropores in the stent body or mixed or bound to a polymer coating applied on stent, to inhibit neointimal tissue proliferation and thereby prevent restenosis. This invention given either systemically or locally also facilitates the performance of the stent in inhibiting restenosis.

### BACKGROUND OF THE INVENTION

### Restenosis limits PTCA success in revascularizing atherosclerotic blood vessels.

Atherosclerotic lesions which limit or obstruct coronary blood flow, are the major cause of ischemic heart disease related mortality, resulting in 500,000-600,000 deaths annually. Percutaneous translumenal coronary angioplasty (PTCA) to open the obstructed artery was performed in over 550,000 patients in the U.S. and 945,000+ patients worldwide in 1996 (Lemaitre et al., 1996). A major limitation of this technique is the problem of post-PTCA closure of the vessel, both immediately after PTCA (acute occlusion) and in the long term (restenosis): 30% of patients with subtotal lesions and 50% of patients with chronic total lesions will go on to restenosis after angioplasty. Additionally, restenosis is a significant problem in patients undergoing saphenous vein bypass graft. The mechanism of acute occlusion appears to involve several factors and may result from vascular recoil with resultant closure of the artery and/or deposition of blood platelets along the damaged length of the newly opened blood vessel followed by formation of a fibrin/red blood cell thrombus. Restenosis after angioplasty is a more gradual process and involves initial formation of a subcritical thrombosis with release from adherent platelets of cell derived growth factors with subsequent proliferation of intimal smooth muscle cells resulting in vascular hyperplasia. It is important to note that both thrombosis and myointimal cell proliferation contribute to the restenotic process.

### Restenosis represents a significant treatment problem

In the U.S., a 30 - 50% restenosis rate translates to 120,000 - 200,000 U.S. patients at risk of restenosis. If only 80% of such patients elect repeat angioplasty (with the remaining 20% electing coronary artery bypass graft) is added to the cost of coronary artery bypass graft for the remaining 20%, the total cost for restenosis can be estimated to be in the billions of dollars. Thus, successful prevention of restenosis could result not only in significant therapeutic benefit but also in significant health care savings.

### Restenosis is a multifactorial process

While the exact mechanism for restenosis is still uncertain, the general aspects of the restenosis process have been identified:
- In the normal arterial wall, smooth muscle cells (SMC) proliferate at a low rate (<O.I%/day; ref). SMC in vessel wall exists in a 'contractile' phenotype characterized by 80-90% of the cell cytoplasmic volume occupied with the contractile apparatus. Endoplasmic reticulum, Golgi, and free ribosomes are few and located in the perinuclear region. Extracellular matrix surrounds SMC and is rich in heparin-like glycosylaminoglycans which are believed to be responsible for maintaining SMC in the contractile phenotypic state (Campbell and Campbell, 1985).
- Upon pressure expansion of an intracoronary balloon catheter during angioplasty, smooth muscle cells within the arterial wall become injured, initiating a thrombotic and inflammatory response. Cell derived growth factors such as platelet derived growth factor (PDGF), basic fibroblast growth factor (bFGF), epidermal growth factor (EGF), thrombin, etc. released from platelets (i.e., PDGF) adhering to the DAMAGED arterial luminal surface, invading macrophages and/or leukocytes, or directly from SMC (i.e., bFGF) provoke a proliferation and migratory response in medial SMC. These cells undergo a phenotypic change from the contractile phenotyope to a 'synthetic' phenotype characterized by only few contractile filament bundles but extensive rough endoplasmic reticulum, Golgi and free ribosomes. Proliferation/migration usually begins within 1-2 days post-injury and peaks at 2 days in the media, rapidly declining THEREAFTER (Campbell and Campbell, 1987; Clowes and Schwartz, 1985).
- Daughter synthetic cells migrate to the intimal layer of arterial smooth muscle and continue to proliferate and begin to secrete significant amounts of extracellular matrix proteins. Proliferation and migration continues until the damaged luminal endothelial layer regenerates at which time proliferation ceases within the intima, usually within 7-14 days postinjury. The remaining increase in intimal thickening which occurs over the next 3-6 months is due to an increase in extracellular matrix rather than cell number. Thus, SMC migration and proliferation is an acute response to vessel injury while intimal hyperplasia is a more chronic response. (Liu *et al*., 1989).

### Restenosis ― Experimental Studies

Numerous agents have been examined for presumed antiproliferative actions in restenosis and have shown some activity in experimental animal models. Some of the agents which have been shown to successfully reduce the extent of intimal hyperplasia in animal models include: heparin and heparin fragments (Clowes and Kamovsky, 265 Nature, 25-626, (1977); Guyton, J.R. et al. 46 Circ. Res., 625-634, (1980); Clowes, A.W. and Clowes, M.M., 52 Lab. Invest., 611-616, (1985); Clowes, A.W. and Clowes, M.M., 58 Circ. Res., 839-845 (1986); Majesky et al., 61 Circ Res., 296-300, (1987); Snow et al., 137 Am. J. Pathol., 313-330 (1990); Okada, T. et al., 25 Neurosurgery, 92-898, (1989) colchicine (Currier, J.W. et al., 80 Circulation, 11-66, (1989), taxol (ref), agiotensin converting enzyme (ACE) inhibitors (Powell, J.S. et al., 245 Science, 186-188 (1989), angiopeptin (Lundergan, C.F. et al., 17 Am. J. Cardiol. (Suppl. B); 132B-136B (1991), Cyclosporin A (Jonasson, L. et. al., 85 Proc. Nati, Acad. Sci., 2303 (1988), goat-anti-rabbit PDGF antibody (Ferns, G.A.A., et al., 253 Science, 1129-1132 (1991), terbinafine (Nemecek, G.M. et al., 248 J. Pharmacol. Exp. Thera., 1167-11747 (1989), trapidil (Liu, M.W. et al., 81 Circulation, 1089-1093 (1990), interferon-gamma (Hansson, G.K and Holm, 84 J. Circulation. 1266-1272 (1991), steroids (Colbum, M.D. et al., 15 J. Vasc. Surg., 510-518 (1992), see also Berk, B.C. et al., 17 J. Am. Coll. Cardiol., 111B-1 17B (1991), ionizing radiation (ref), fusion toxins (ref) antisense oligonucleotides (ref), gene vectors (ref), and cladribine(see below). Antiproliferative action on SMC in vitro has been demonstrated for many of these agents, including heparin and heparin conjugates, taxol, colchicine, ACE inhibitors, fusion toxins, antisense oligonucleotides and ionizing radiation. Thus, agents with antiproliferative activity may have therapeutic utility in reducing intimal hyperplasia.

### Restenosis ― Clinical Studies

However, unlike attempts in animal models, attempts in human angioplasty patients to prevent restenosis by systemic pharmacologic means have thus far been unsuccessful. Neither aspirin-dipyridamole, ticlopidine, anticoagulant therapy (acute heparin, chronic warfarin, hirudin or hirulog), thromboxane receptor antagonism nor steroids have been effective in preventing restenosis although platelet inhibitors have been effective in preventing acute reocclusion after angioplasty (Mak and Topol, 1997; Lang *et al.,* 1991; Popma *et al.,* 1991). Additionally, the 7E3 humanized monoclonal antibody fragment to the platelet GP IIb/IIIa receptor is still under study but has not shown promising results for the reduction in restenosis following angioplasty and stenting (). Other agents which have also been unsuccessful in the prevention of restenosis include the calcium channel antagonists, prostacyclin mimetics, angiotensin converting enzyme inhibitors, serotonin receptor antagonists, and antiproliferative agents. These agents must be given systemically, however, and attainment of a therapeutically effective dose may not be possible; antiproliferative (or anti-restenosis) concentrations may exceed the known toxic concentrations of these agents so that levels sufficient to produce smooth muscle inhibition may not be reached (Mak and Topol, 1997; Lang *et al*., 1991; Popma *et al*., 1991).

Additional clinical trials in which the effectiveness for preventing restenosis of dietary fish oil supplements or cholesterol lowering agents has been examined have shown either conflicting or negative results so that no pharmacological agents are as yet clinically available to prevent post-angioplasty restenosis (Mak and Topol, 1997; Franklin and Faxon, 1993; Serruys, P.W. *et al*., 1993). Recent observations suggest that the antilipid/antioxident agent, probucol may be useful in preventing restenosis but this work requires confirmation (Tardif et al., 1997; Yokoi, et al., 1997). Probucol is presently not approved for use in the United States and a 30 day pretreatment period would preclude its use in emergency angioplasty. Additionally, application of ionizing radiation has shown some promise in reducing or preventing restenosis after angioplasty in patients with stents (Teirstein et al., 1997). Currently, however, the most effective treatments for restenosis is repeat angioplasty, atherectomy or coronary artery bypass graft, because no therapeutic agents currently have US Federal Regulatory Agency (USFDA) regulatory approval for use for the prevention of post-angioplasty restenosis.

### Stents and restenosis

Unlike systemic pharmacologic therapy, stents have proven useful in partially preventing restenosis. Stents, such as seen in layout in Figure 4, are balloon-expandable slotted metal tubes (usually, but not limited to, stainless steel), which when expanded within the lumen of an angioplastied coronary artery, provide structural support to the arterial wall. This support is helpful in maintaining an open path for blood flow. In two randomized clinical trials, stents increased angiographic success after PTCA, increased the stenosed blood vessel lumen and reduced (but did not eliminate) the incidence of restenosis at 6 months (Serruys et al., 1994; Fischman et al., 1994).

Additionally, in a preliminary trial, heparin coated stents appear to possess the same benefit of reduction in stenosis diameter at follow-up as was observed with non-heparin coated stents. Heparin coating also appears to have the added benefit of producing a reduction in sub-acute thrombosis after stent implantation (Serruys et al., 1996). Thus, 1) sustained mechanical expansion of a stenosed coronary artery with a stent has been shown to provide some measure of restenosis prevention, and 2) coating of stents with heparin has demonstrated both the feasibility and the clinical usefulness of delivering drugs locally, at the site of injured tissue.

### Cladribine for the prevention of restenosis.

Cladribine (2-CdA) is the 2-chloro-2'-deoxy derivative of the purine nucleoside, adenosine. 2-CdA is resistant to degradation by adenosine deaminase, one of two intracellular adenine nucleotide regulatory enzymes, found in most cells. The other enzyme, 5'-nucleotidase, is present in variable amounts in different cell types (Carson et al., 1983). After initial phosphorylation to its monophosphate derivative by the intracellular enzyme, deoxycytidine kinase, 2-CdA is converted to a 5'-triphosphate (2-CdATP) which accumulates in levels which may be 50-fold greater than normal dATP levels. Thus, in cells such as leukocytes, which contain a high ratio (>0.04) of deoxycytidine kinase to 5'-nucleotidase, 2-CdA and its subsequent metabolites will tend to accumulate in pharmacological concentrations (Carson et al., 1983). Such high levels of a nucleoside triphosphate are known to inhibit the enzyme ribonucleotide reductase in rapidly dividing cells, thus preventing synthesis of deoxynucleotides required for DNA synthesis.

In resting cells, 2-CdATP is incorporated into DNA which results in single strand breaks. Breaks in DNA results in the activation of poly (ADP-ribose) polymerase which in tum leads to a depletion of NAD, ATP and a disruption of cell metabolism (Carson et al., 1986; Seto et al., 1985). Further activation of a Ca²⁺/Mg²⁺-dependent endonuclease results in cleavage of the DAMAGED DNA into fragments leading to programmed cell death (apoptosis). Thus, 2CdA can be cytotoxic to both resting and dividing cells (Beutler, 1992). The cytotoxic action of cladribine has been shown for both leukocytes and monocytes (Carrera et al., J. Clin. Invest. 86:1480-1488, 1990; Carson, D.A., et al., Blood 62:737-743, 1983), cell types known to play a role in the inflammatory process which accompanies restenosis. Additionally, data presented herein demonstrate that cladribine also possesses an ability to inhibit smooth muscle cell proliferation, an action previously unknown for cladribine (see Example 1). Therefore, cladribine may possess a unique spectrum of therapeutic action comprising, 1) prevention of the leukocyte accumulation known to occur at sites of arterial injury and inflammation as well as 2) the prevention of smooth muscle hyperplasia which results from angioplasty and stent implantation.

### SUMMARY OF THE INVENTION

The current invention comprises an approach to solving the clinical problem of restenosis, which involves the administration of the antineoplastic agent, cladribine, to patients undergoing PTCA or stent implantation. In one embodiment of the invention, cladribine is administered to patients systemically, either subcutaneously, intramuscular or intravenously. A therapeutic effect could be achieved with, but not limited to, a dose of 90 ug/kg/day for 7 days by continuous intravenous infusion. Similarly, a therapeutic effect could be achieved with, but not limited to, a dose of 140 ug/kg/day for 5 days by subcutaneous administration.

In another embodiment of the invention, cladribine is bound to the surface of a stent by means of incorporation within either a biodegradable or biostable polymeric coating. Alternatively, cladribine could be incorporated into a stent constructed with a grooved reservoir. Stents are metallic slotted tubular devices which, 1) provide structural support for arteries which become dilated and injured during the process of angioplasty, and 2) at least partially limit the extent of restenosis after angioplasty. Thus, delivery of a cladribine-containing stent to a coronary artery injured during the process of angioplasty would provide the added therapeutic benefit of limiting the degree of local smooth muscle cell proliferation, enhancing the restenosis-limiting action of the stent.

### DETAILED DESCRIPTION OF THE DRAWINGS:

The invention will be better understood in connection with the following figures in which:

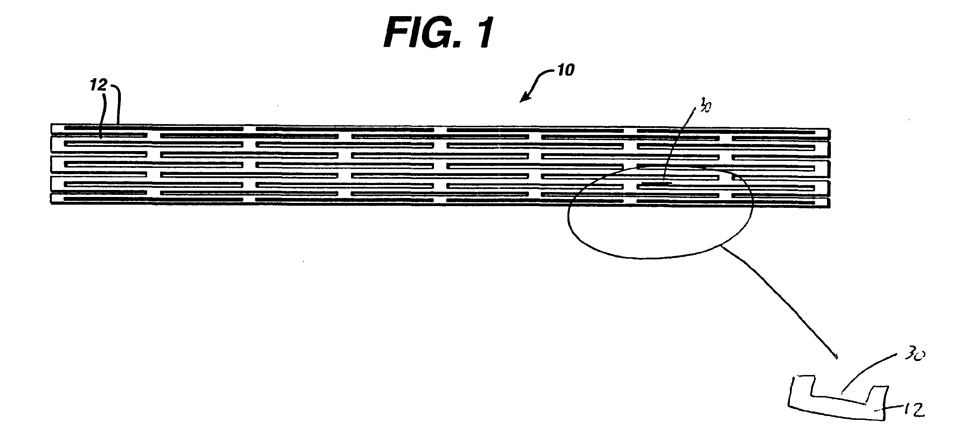

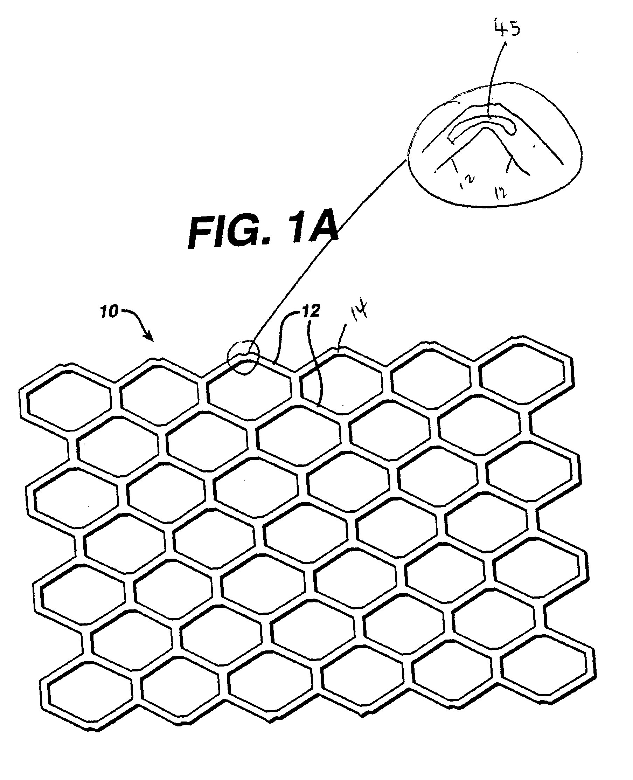

Figures 1 and 1A are top views and section views of a stent containing reservoirs as described in the present invention.

### Detailed Description of the Invention

As stated previously, implantation of a coronary stent in conjunction with balloon angioplasty is highly effective in treating acute vessel closure and may reduce the risk of restenosis. Intravascular ultrasound studies (Mintz et al., 1996) suggest that coronary stenting effectively prevents vessel constriction and that most of the late luminal loss after stent implantation is due to plaque growth, probably related to neointimal hyperplasia. The late luminal loss after coronary stenting is almost two times higher than that observed after conventional balloon angioplasty. Thus, inasmuch as stents prevent at least a portion of the restenosis process, an agent which prevents inflammation and the proliferation of SMC combined with a stent may provide the most efficacious treatment for post-angioplasty restenosis (Bauters et al., 1996). In this regard, a stent in conjunction with systemic cladribine treatment or local delivery of cladribine is an attractive treatment. Either systemic or local delivery of cladribine from a stent has the following advantages:
- prevention of vessel constriction with the stent;
- prevention of leukocyte and monocyte accumulation and smooth muscle cell proliferation at the site of vascular injury with cladribine

Local cladribine administration to stented coronary arteries might have additional therapeutic benefit:
- higher tissue concentrations would be achievable than would occur with systemic administration
- reduced systemic toxicity
- single treatment/ease of administration

As seen in the figures it is possible to modify currently manufactured stents in order to adequately provide the drug dosages such as cladribine. As seen in Figure 1, any stent 10 having strut 12, can be modified to have a certain reservoir 30. Each of these reservoirs can be "open" or "closed" as desired. These reservoirs can hold the drug to be delivered. Figure 1a shows a stent 10 with a reservoir 45 created at the apex 14 of struts 12. Of course, this reservoir 45 is intended to be useful to deliver cladribine or any other drug at a specific point of flexibility of the stent. Accordingly, this concept can be useful for "second" or "third" generation-type stents.

In any of the foregoing devices, however, it is useful to have the drug dosage applied with enough specificity and enough concentration to provide an effective dosage in the lesion area. In this regard, the reservoir size in the stent struts must be kept at a size of about 0.1 mm to about 1 mm depth, and 7 mm to 15 mm length, or enough to hold at least a therapeutic amount of the drug. Then, it should be possible to adequately apply the drug dosage at the desired location and in the desired amount.

These and other concepts will are disclosed herein. It would be apparent to the reader that modifications are possible to the stent or the drug dosage applied. In any event, however, the any obvious modifications should be perceived to fall within the scope of the invention which is to be realized from the attached claims and their equivalents.

### EXAMPLE 1

To assess the ability of cladribine to prevent cell proliferation, human smooth muscle or endothelial cells (Clonetics, Walkersville, MD) were seeded at a density of 2000 cells/cm² (approximately 3600 cells/well) into each well of 12-well plates and cultured with 1.5 ml of growth medium containing 5% fetal calf serum (FCS). After 24 hours, the growth medium was changed and fresh medium containing 10 ng/ml platelet-derived growth factor AB (PDGF AB; LIFE Technologies), as well as various concentrations of cladribine (0.001 - 10,000 nM) were added with triplicate wells. Medium was replaced with fresh cladribine-containing medium after 3 days. On day six, cells were detached by trypsinization to yield a cell suspension, lightly centrifuged to pellet and then counted manually using a Neubauer hemacytometer system. Clee viability was assessed by trypan blue exclusion.

Table 1 provides the percent inhibition of the various tested concentrations of cladribine on human smooth muscle and endothelial cells in culture. Cladribine produced a concentration-related decrease in the proliferation of both smooth muscle and endothelial cells in this model system. IC₅₀ values (concentration required to produce a reduction in proliferation to 50% of the vehicle-treated cell count) for the inhibition of smooth muscle cell and endothelial cell growth were 23 nM and 40 nM, respectively. Cladribine was thus approximately twice as potent as an inhibitor of smooth muscle cells as it was as an inhibitor of endothelial cells. Both IC₅₀ values are within the range of inhibitory concentrations reported for cladribine on human monocytes (Carrera et al., J. Clin. Invest. 86:1480-1488, 1990) and normal bone marrow, lymphocytic and lymphoblastic cell lines (Carson, D.A. et al., Blood 62: 737-743, 1983). Thus, concentrations of cladribine known to be effective at inhibiting peripheral leukemic blood cell proliferation and bone marrow cells are also effective at inhibiting proliferating vascular smooth muscle and endothelial cells. Cladribine may therefore be therapeutically useful for inhibition of the intimal smooth muscle cell proliferation which accompanies stent implantation.

**TABLE 1.**

| Inhibition of human vascular cell proliferation with cladribine. | | | | | | | | | | |
|---|---|---|---|---|---|---|---|---|---|---|
| | Cladribine (nM) | | | | | | | | | |
| | Control | Vehicle | 0.001 | 0.01 | 0.1 | 1 | 10 | 100 | 1000 | 10,000 |
| SMC | 100 | 108 | - | 104 | 86 | 85 | 54 | 58 | 12 | -4 |
| EC | 100 | 100 | 100 | 90 | 79 | 75 | 59 | 57 | 35 | 10 |
| Values represent % of PDGF-stimulated increase in cell count. Each % is the mean of triplicate determinations. SMC, smooth muscle cells; EC, endothelial cells. | | | | | | | | | | |

## Claims

1. In combination:
a stent for the delivery of drugs to a lumen of a patient; and
a therapeutic dosage amount of cladribine coated to said stent.

2. A stent comprising:
a plurality of struts, said struts expansible within the lumen of the body, and at least one of said struts containing a reservoir therein said reservoir filled with a therapeutic dosage amount of cladribine.

3. The use of cladribine in the manufacture of a medicament for the prevention or treatment of restenosis in a patient, wherein said cladribine is administered to said patient subcutaneously, intramuscularly, intravenously, or on the strut of a stent, preferably in a dosage of at least 40nM.
